# EUROPEAN PATENT SPECIFICATION

(11) **EP 2 184 024 B1**
(45) Date of publication and mention of the grant of the patent: **09.01.2019**
(21) Application number: 08828345.2
(22) Date of filing: 27.08.2008
(51) Int. Cl.: A61B 18/12

(54) **MEDICAL DEVICE**
MEDIZINISCHE VORRICHTUNG
DISPOSITIF MÉDICAL

(30) Priority: 28.08.2007 JP 2007221901; 26.03.2008 JP 2008081424
(43) Date of publication of application: 12.05.2010
(73) Proprietor: Terumo Kabushiki Kaisha, Tokyo 151-0072 (JP)
(72) Inventor: KATOU, Yukitoshi, Ashigarakami-gun Kanagawa 259-0151 (JP)
(74) Representative: Dossmann, Gérard
(86) International application number: PCT/JP2008/065280
(87) International publication number: WO 2009/028542

(56) References cited:
- WO-A1-2007/100067
- WO-A2-2006/110830
- JP-A- 2000 210 301
- JP-A- 2006 521 181
- JP-U- 59 177 411
- US-A1- 2004 243 122
- US-A1- 2004 243 122
- US-A1- 2006 271 089
- US-A1- 2007 055 333
- US-A1- 2007 106 290
- US-A1- 2007 112 347
- US-A1- 2007 123 851
- US-A1- 2007 150 000

## Description

### TECHNICAL FIELD

The present invention relates to a medical device used for a medical treatment or the like which closes a defect occurring in a living body.

### BACKGROUND ART

Recently, as a medical treatment device with respect to a patent foramen ovale (hereinafter, referred to as PFO: Patent Foramen ovale) which is a cardiogenic factor of a stroke and a hemi headache, there has been proposed a device described in WO2004/086944.

This PFO closing device is a device in which an apparatus is inserted into the foramen ovale from the right atrium toward the left atrium, a foramen ovale valve is pulled to the foramen ovale so as to close it and the biological tissue is to be fused by applying electric energy.

However, the foramen ovale, the foramen ovale valve and the atrial septum secundum are different not only in small & large sizes but also in a state of thicknesses, shapes or the like depending on persons and according to circumstances, the size or the like of the apparatus is restricted a lot. Also, even on an occasion when the procedure is performed, there is a fear that it becomes difficult to pull various forms of foramen ovale valves to the foramen ovale at anytime and reliably.

Consequently, the present patent applicant proposed previously a PFO closing device in which the foramen ovale valve and the atrial septum secundum are sandwiched by a pair of electrodes and the biological tissue is fused reliably by applying electric energy from both the electrodes (see WO2007/100067).

This device uses clamping means in which one side thereof is made of a sticking member composed of a needle electrode and the other side thereof is made of a sandwiching member for sandwiching the foramen ovale valve and the atrial septum secundum with respect to the sticking member and the sticking member is stuck into the foramen ovale valve and thereafter, the foramen ovale valve and the atrial septum secundum are sandwiched with respect to the sandwich member which is the other electrode, the biological tissue is applied with electric energy, and the fusion is carried out.

This device can be used also in case of closing defects such as a congenital atrial septum secundum defect (ASD), a PFO, a ventricular septal defect (VSD) and a patent ductus arteriosus (PDA), and it is a device having high general versatility and in particular, foreign substances are not indwelled in the body, the constitution becomes simple, also the procedure becomes easy and the foramen ovale valve and the atrial septum secundum can be fused reliably.

However, in case of steering this device, a cable for supplying electric energy and a fine and long-line shaped member for steering the clamping means remotely are provided on the steering unit at hand, so that various steering means exist in the steering unit at hand and there is a fear of miss-steering. In particular, in case of sandwiching a tissue and thereafter fusing it by applying electric energy and when the electric energy is always applied, there is a fear that electric current flows not only in the desired region but also in an unnecessary region and there is also a fear that a thrombus will be attached depending on the temperature-rise of the sticking member or the like. When the thrombus is exfoliated from the sticking member or the like and reaches a peripheral vessel of the brain from the left atrium, a cerebral infarction or the like would occur, so that the attachment of the thrombus should be prevented as much as possible.

Also, in case of sandwiching a biological tissue by clamping means, the surgery operator sticks a sticking member to the foramen ovale valve while maintaining a state in which the foramen ovale valve which has a thin-film shape and is easily deformed is held by a sandwiching member. Specifically, for example, in a state in which the sandwiching member is held by the left hand so as not to be displaced, the sticking member is steered by the right hand and the sticking is executed, but when the holding by the left hand is loosened, the sticking cannot be executed and therefore, there is also such a practical problem that the operator must concentrate on both the right and left hands.

US 2007/0123851 A1 discloses a medical device according to the preamble of claim 1.

### DISCLOSURE OF THE INVENTION

### Problem to be solved by the Invention

A purpose of the present invention lies in providing a medical device in which it is possible to perform a sandwiching operation of a biological tissue by clamping means and a supply operation of electric energy independently, it is possible to reliably prevent a thrombus attachment caused by the temperature-rise of the sticking member or the like, which is caused by a fact that unnecessary electric current flows, the constitution of the steering unit at hand is simplified, and it is possible to perform a surgical procedure smoothly, safely and reliably.

### Means for solving the Problem

The invention provides a medical device according to claim 1. Preferred embodiments are defined in the dependent claims. The methods disclosed herein do not form part of the invention. The same applies to all other embodiments, aspects and examples herein that do not fall under the scope of the claims.

A medical device of the present invention which achieves aforesaid object is characterized by including: clamping means including a sticking member for sticking a biological tissue which lies at a periphery of a defect existing in a biological tissue and a sandwiching member for sandwiching aforesaid biological tissue so as to close aforesaid defect by cooperating with aforesaid sticking member; a catheter into which aforesaid sticking member and aforesaid sandwiching member are inserted; electric energy supply means for supplying electric energy to aforesaid sticking member and aforesaid sandwiching member of aforesaid clamping means; and a steering unit at hand mounted with a proximal portion of aforesaid catheter for steering aforesaid clamping means, and a medical device characterized by closing aforesaid defect by supplying electric energy from aforesaid electric energy supply means and by joining aforesaid biological tissues mutually in a state of sandwiching aforesaid biological tissue by aforesaid sticking member and aforesaid sandwiching member, in which aforesaid steering unit at hand includes: contact members which are connected to the other terminal portions of conductive wires whose one terminals are connected to aforesaid electric energy supply means and which are provided at mutually separated positions, terminals respectively provided at respective steering-members which move said sticking member and said sandwiching member and which are connected electrically to aforesaid contact member, and a control unit for on-off controlling the supply of aforesaid electric energy, wherein aforesaid respective terminals contact with aforesaid contact members respectively along with the move of aforesaid respective steering-members and also, it is constituted so as to supply aforesaid electric energy to aforesaid biological tissue between aforesaid sticking member and sandwiching member by turning on aforesaid control unit.

### Effect of the Invention

It is possible for the present invention constituted in this manner to supply electric energy after the sandwiching state of the biological tissue is established by the steering of the clamping means, so that it is possible to perform a surgical procedure smoothly, safely and reliably, and it is possible to provide a medical device in which a thrombus attachment caused by temperature-rise along with the electric energy supply to an unnecessary region or at an unnecessary time point can be prevented reliably.

If aforesaid control unit is constituted by a switch provided on the steering unit at hand or in a vicinity thereof and the attachment and detachment between a steering-lever for steering the clamping means and a connection member on the side of the electric energy supply means, it is possible for the electric energy supply to be carried out after a biological tissue is sandwiched reliably between a sticking member and a sandwiching member. In other words, if it is in a state in which the sticking member does not stick the biological tissue, the electric energy is never supplied, unnecessary electric energy supply never happens and the electric energy supply starts for the first time after the clamping means holds and presses the biological tissue, so that it is possible to prevent miss-steering of the surgical procedure and to perform a surgical procedure smoothly, safely and reliably, and the safety of the device becomes extremely high.

In a case in which aforesaid contact member and terminal contact and if the terminal and the contact member contact before reaching the moving completed position of aforesaid terminal, even if a sandwiching state of the sandwiching member changes depending on the sticking state of the sticking member, which is different from person to person or depending on the personal difference of the thickness of the atrial septum secundum, the electrical conduction becomes possible by adjusting the conduction position of the terminal or the contact member corresponding to this change and reliability of the surgical procedure is secured.

In particular, the respective terminals contact with the contact member along with the movement of the respective steering-members of the sticking member and the sandwiching member, so that the steering of the sticking member and the sandwiching member on the steering unit at hand and an electrical conductable state become in a linked relation reliably, and it does not become in an electrical conduction state unless a predetermined surgical procedure is completed and safety is secured also with respect to the sequence in the surgical procedure. However, either one of the groups of the contact members and the terminals which exist by two pairs and which contact with each other may be allowed to be always in a contact state.

If aforesaid contact member is constituted by a tubular collar having an attachment portion, a leg portion and a spring, it happens that aforesaid terminal will contact with the terminal before reaching the moving completed position and therefore, by a simple constitution, it is possible to have correspondence with various biological tissues which are all different depending on persons, and it is possible to attempt to realize simpleness of the constitution.

With respect to aforesaid steering unit at hand, if respective steering-levers for the sticking member and for the sandwiching member are provided slidably at the main body portion which is grippable by one hand, it is possible to perform the surgical procedure by the steering of the respective steering-levers and it is possible to attempt to realize smoothness, safety and reliability of the surgical procedure.

With respect to aforesaid steering unit at hand, if the sticking steering-lever is protruded to one surface of the flat main body portion and the steering-lever for the sandwiching member is protruded to the other surface side, miss-steering of the respective steering-levers disappears and it is possible to attempt to realize smoothness, safety and reliability of the surgical procedure.

If aforesaid steering unit at hand is constituted by the main body portion and a slide portion provided so as to be approached and separated with respect to aforesaid main body portion, if either one of aforesaid sticking and sandwiching steering-levers is provided on the upper surface of aforesaid main body portion and if the other steering is carried out by aforesaid slide portion, it happens that one of the steering-levers lies on the upper surface of the main body portion and the other steering is to be carried out by the slide portion which is approached and separated with respect to the main body portion, so that it is possible to prevent miss-steering of the surgical procedure and to perform the surgical procedure smoothly, safely and reliably.

If there is provided in aforesaid medical device with a holding portion for holding the biological tissue non-retractably with respect to the sticking direction of the sticking member, the sticking of the sticking member becomes easy, it is possible to have correspondence with various biological tissues which are all different depending on persons and it is possible to attempt to realize speediness of the surgical procedure.

If there is provided in aforesaid medical device with positioning hold means composed of a positioning portion for positioning the sticking member and a holding portion for holding the biological tissue non-retractably with respect to the sticking direction of aforesaid sticking member, it is possible, by the positioning portion, to position the sticking member accurately with respect to the biological tissue having a defect, the sticking of the sticking member becomes easy by the holding portion, it is possible to have correspondence with various biological tissues which are all different depending on persons, and accuracy of the surgical procedure is improved furthermore.

Aforesaid positioning portion displaces an elastic member outward by steering a long main operation rod advancingly and retractingly in the axis direction in a main tube provided advanceably and retractably in a catheter and if aforesaid elastic member positions the sticking member at a central portion of aforesaid defect by attaching it at an inner edge of the defect of the biological tissue elastically, the positioning of the sticking member becomes possible only by steering the main operation rod advancingly and retractingly in the axis direction.

If it is constituted with respect to aforesaid holding portion such that the distal portion of aforesaid main operation rod is curved and the biological tissue is held by steering a long main operation rod advancingly and retractingly in the axis direction in a main tube provided advanceably and retractably in a catheter, the holding of the biological tissue become possible only by steering the main operation rod advancingly and retractingly in the axis direction.

If the long main operation rod provided advanceably and retractably in the axis direction in the main tube of the medical device is made to be rotatable as much as 360 degrees centering around the axis line in the main tube, it is possible to insert the distal end of the device through the defect of the biological tissue easily regardless of the state of the biological tissue and it is possible to attempt easiness and speediness of a surgical procedure.

In the steering unit at hand when steering by a single hand, if the state in which the sticking member is protruded from a distal portion of a catheter is locked by a lock mechanism, the sticking state of the sticking member is maintained reliably, so that it is possible to perform the surgical procedure accurately and also smoothly.

In aforesaid lock mechanism, if a bump member which restricts the slide move of the sticking steering-lever is provided position-fixedly on the distal side of a slide groove which is formed at the main body portion and in which the sticking steering-lever slides, the locking is realized by an uncomplicated and simple mechanism, the steering unit at hand never becomes large-scaled and a device having excellent steerability can be realized.

If there is employed a constitution with respect to the steering unit at hand in which a holding-portion locking mechanism for position-fixing the main operation rod is provided at a slide portion provided freely advancingly and retractingly with respect to the main body portion, and the sandwiching of aforesaid sandwiching member and the contact with aforesaid contact member of aforesaid terminal are to be performed in a lump by advancing and retracting aforesaid slide portion with respect to aforesaid main body portion, the steering becomes easy and it is possible to attempt simpleness of the surgical procedure.

If there is employed a constitution in which aforesaid holding portion includes a curving mechanism for curving the distal portion of aforesaid main operation rod by steering aforesaid main operation rod advancingly and retractingly in the axis direction and with respect to this curving mechanism, a distal sleeve body mounted at the distal end of aforesaid main operation rod and aforesaid main tube are interlocked by a first elastic wire and a second elastic wire, it is possible to perform aforesaid positioning and holding operations concurrently and furthermore, simpleness of the constitution is achieved and also the surgical procedure becomes easy.

If a distal end of a guiding catheter for guiding aforesaid catheter is curved, it is easier for aforesaid catheter, regardless of states of the foramen ovale valve and the atrial septum secundum which are different depending on persons, to go toward the foramen ovale between the foramen ovale valve and the atrial septum secundum compared with a case of a straight shape, and safety, usability and speediness of the surgical procedure are improved.

Still other purpose, feature and characteristic of the present invention will become clear in view of preferable exemplified embodiments which will be illustrated by the explanation and the attached drawings hereinafter.

### BEST MODE FOR CARRYING OUT THE INVENTION

Hereinafter, exemplified embodiments of the present invention will be explained in detail with reference to the drawings.

### <First Exemplified Embodiment>

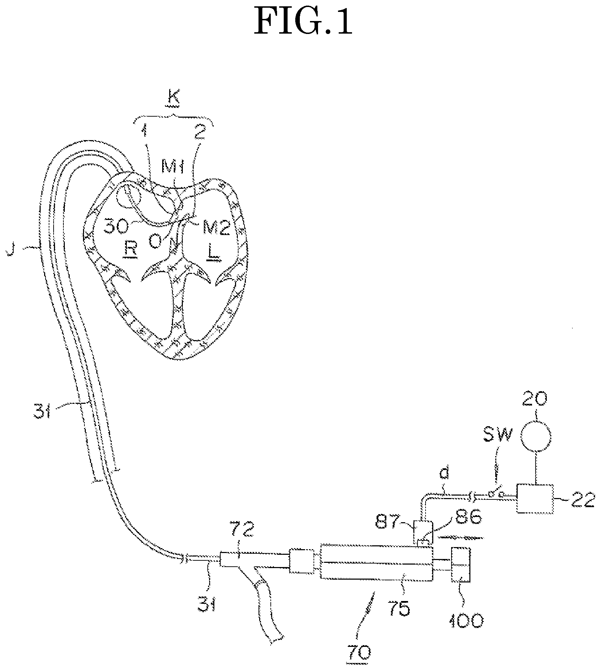

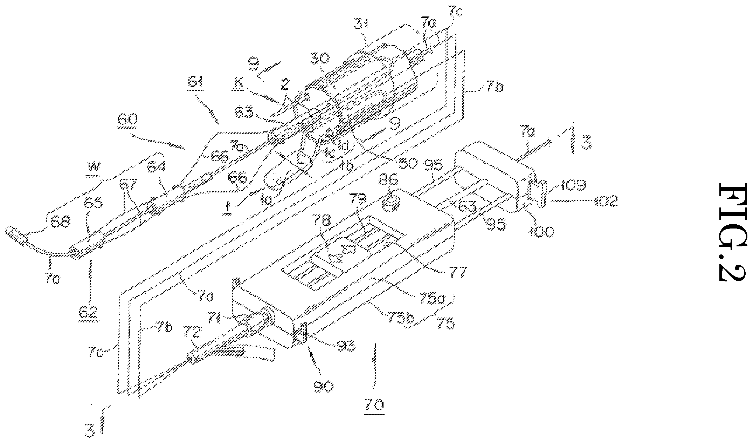

First, it will be outlined with respect to a medical device of the present exemplified embodiment. This device is a device which is used mainly for closing PFO and, as shown in FIGS. 1, 2, includes a steering unit at hand 70 provided on the proximal side, a guiding catheter 31 whose proximal end is mounted on the steering unit at hand 70, a catheter 30 provided in the guiding catheter 31, clamping means K which is provided at a distal portion of the catheter 30 and which sandwiches a foramen ovale valve M2 and an atrial septum secundum M1, energy supply means 20 for supplying energy which fuses or joins a biological tissue M (generic term of M1, M2) of a portion sandwiched by the clamping means K, and positioning hold means 60 for performing the surgical procedure by the clamping means K stably and also accurately. It should be noted in the following explanation that the side of the steering unit at hand of the device is referred to as "proximal side" and the side of the clamping means K is referred to as "distal side". Also, in FIG. 2, only the steering unit at hand 70 is drawn by a state of being demagnified because of space limitations.

This device, when being used, first, is inserted, for example, from a femoral venous J in a state in which there is housed, in a guiding catheter 31, the whole clamping means K which is provided at the distal end of the catheter 30. If the distal end reaches the region of the heart at which the procedure is executed, the clamping means K is protruded from the distal end of the guiding catheter 31 and there are sandwiched, by the clamping means K, the tissues of the atrial septum secundum M1 and the foramen ovale valve M2 of the heart having the defect O of the foramen ovale. In this sandwich state, the clamping means K is supplied with electric energy, both the tissues are heated and fused and the defect O is closed. It should be noted in the drawing that "L" denotes a left atrium and "R" denotes a right atrium.

It will be described in more detail. The clamping means K is constituted, as shown in FIG. 2, by a sandwich member 1 directly contacting with one side surface of the atrial septum secundum M1 and a sticking member 2 which is stuck into the foramen ovale valve M2. The sandwich member 1 and the sticking member 2 function as electrode members respectively, and the proximal portions thereof are held by a holder 50 provided at the distal end of the catheter 30 and it is constituted such that they take positions to face to each other when they are projected from the holder 50.

The sandwich member 1 is constituted by a flat board portion 1a including generally a flat plate shape and a predetermined width L, and a pair of wire portions 1b connected to the proximal portion; and one piece of steering-member 7b is connected on the proximal side of the wire portion 1b which is formed in a U-shape. When protruding the steering-member 7b in the axial direction, the sandwich member 1 is protruded from the distal end of the catheter 30 and when retreating the steering-member 7b to the axial direction, it is displaced so as to approach toward the stick portion 2 side.

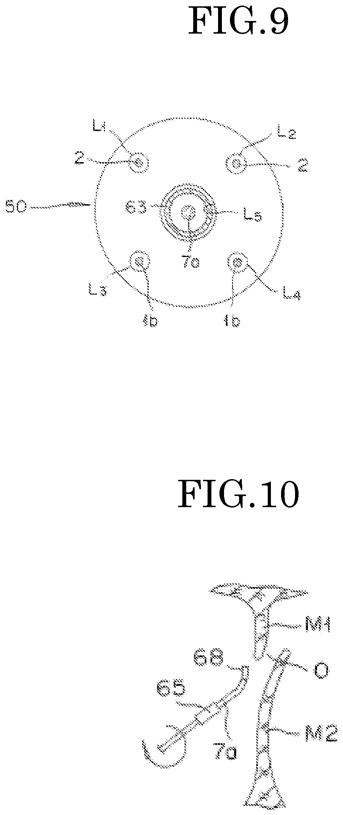

The wire portion 1b includes a bend portion 1c and a straight-shaped portion 1d, the straight-shaped portion 1d is inserted into lumens L3, L4 (see FIG. 9) of the holder 50 so as to be movable forward and backward, so that if the steering-member 7a is traction-operated, it is possible, when the bend portion 1c gets into the entrance portion of the lumens L3, L4 of the holder 50, to displace the sandwich member 1 so as to approach and separate with respect to the sticking member 2 and it is possible to carry out the sandwich of the biological tissue by both the electrode members easily and smoothly even in case of the distal portion of the fine catheter 30.

With respect to the sandwich member 1, a SUS material may be used for a material of the flat board portion 1a thereof, in which it is preferable to use a material which fulfills a function as the electrode and which does not exert bad influence to a living body such as, for example, gold, silver, platinum, tungsten, palladium or alloys including these, Ni-Ti alloy, titanium alloy and the like.

The sticking member 2 is held by lumens L1, L2 formed in the holder 50 so as to be movable forward and backward and the distal portion thereof is constituted so as to be retractable from the holder 50 by operating a steering-member 7c connected to the proximal side.

The sticking member 2 has, as shown in FIG. 2, a structure in which very fine two pieces of needle shaped members whose cross-sections perpendicular to the axes are circles and whose distal ends are sharply pointed are arranged by being mutually separately apart and if the foramen ovale valve M2 is stuck into by such a sticking member 2, it is possible, even for various forms of the foramen ovale valve and the atrial septum secundum, to execute positioning of at least one electrode member to the foramen ovale valve M2 and the sandwich operation of the biological tissue M becomes easy.

It is not always necessary for the sticking member 2 to be a solid cylindrical needle member and it is allowed to Employ a hollow cylindrical shaped member and it is preferable for the outer diameter thereof to be around 0.1mm to 2mm in order to be installed in the catheter 30. For a material, a SUS material is used, but it is also possible to use a material which does not exert bad influence to a living body such as, for example, gold, silver, platinum, tungsten, palladium, titanium or alloys including these, Ni-Ti alloy and the like. Although it is not limited in particular, it is enough if the mutual distance of two pieces of the sticking member 2 is selected such that the foramen ovale valve M2 and the atrial septum secundum M1 can be sandwiched in a certain range, and also with respect to the number of pieces, it may be not only two pieces but also a larger number of pieces than two.

As the steering-members 7b, 7c for moving the sandwich member 1 and the sticking member 2 in/out from the catheter 30, any kinds of members can be employed if they are fine wire-shaped members, can move the clamping means K forward and backward in the catheter 30 and have electric conductivity. For example, it is preferable to use a wire such as stainless, Ni-Ti, titanium and the like. Both the steering-members 7b, 7c are inserted into the catheter 30 and are connected with the energy supply unit 20 through a coupler 87 engaged with a connector portion 86.

In the clamping means K, also the sticking member 2 and also the sandwiching member 1 are in a state in which they are respectively movable independently in the axis line direction with respect to the catheter 30. In this manner, if the sticking member 2 and the sandwiching member 1 are constituted to be movable mutually independently by using the steering-members 7b, 7c, the sticking can be carried out at an arbitrary position in the sticking member 2, the surgical procedure in response to the situation of the biological tissue M becomes extremely easy and smooth, and in the sandwiching member 1, movement or steering for pressing the atrial septum M1 with respect to the foramen ovale valve M2 in a stuck state and positioning of the biological tissue M in the thickness direction become possible.

It should be noted that the holder 50 includes, as shown in FIG. 9, a plurality of lumens L1 to L5, but it is also allowed to constitute these respective lumens L1 to L5 by catheters respectively.

A steering unit at hand 70, as shown in FIG. 1 to FIG. 3, includes a main body portion 75 having a flat hollow box shape and a slide portion 100 provided freely advancingly and retractingly so as to be approached and separated with respect to the main body portion 75.

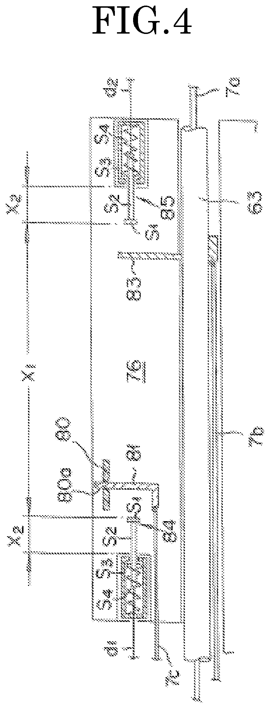

The main body portion 75 is composed of a pair of upper and lower cases 75a, 75b which are aligned face to face; a main tube 63 and the like are passed-through in an internal space 76 (see FIG. 4); and terminals 81, 83, steering-members 7a, 7b, 7c and the like are also housed therein.

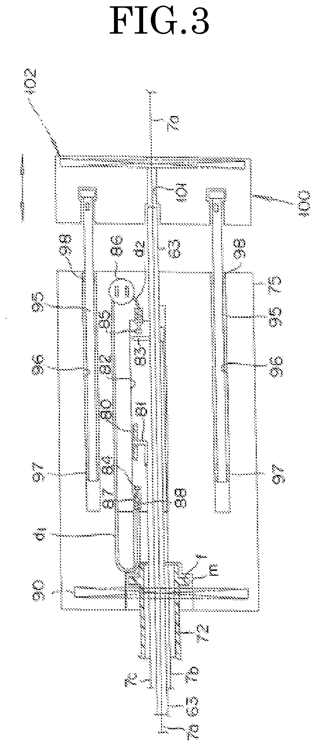

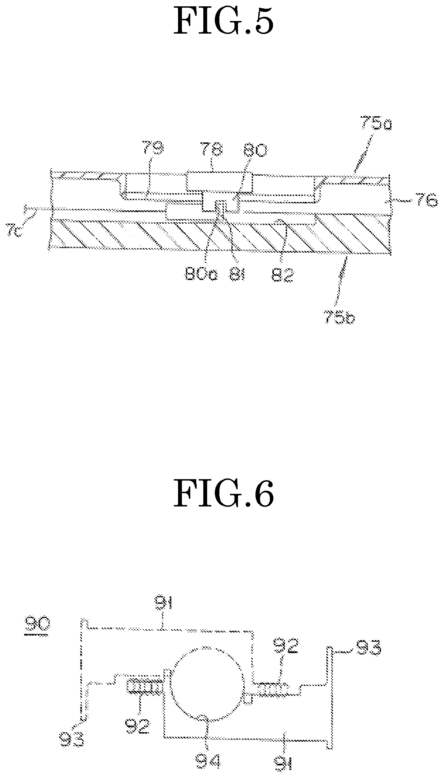

It will be explained in more detail. As shown in FIG. 2, on the surface side (upper surface side) of the upper case 75a, there is formed a comparatively wide concave groove 77 in the center thereof and there is provided here a sticking steering-lever 78 slidably in the longitudinal direction (see white arrows). The sticking steering-lever 78, as shown in FIG. 5, includes a bracket 80, and the bracket 80 is protruded so as to reach the internal space 76 by passing-through a slit 79 (see FIG. 2) formed on the upper case 75a and as shown in FIG. 5, an L-shaped terminal 81 provided at the proximal side of a sticking steering-member 7c is engagement-interlocked to a concave portion 80a formed at the lower end of this bracket 80. Consequently, when the sticking steering-lever 78 is slid along the slit 79, the terminal 81 is, as shown in FIG. 3, slid along a guide groove 82 formed on the lower case 75b and it is constituted such that the sticking member 2 is advanced and retracted through the sticking steering-member 7c.

In the center of the width direction of the upper surface of the lower case 75b, as shown in FIG. 3, there is passed-through a main tube 63 which will be explained in detail later. The main tube 63 is a tube which exerts a sort of center-axis like function of this device, and the proximal side of the main tube 63 is interlocked to the slide portion 100 position-fixedly by an adhesive agent or the like and slides by being guided by both the cases 75a, 75b in response to the slide action of the slide portion 100.

On the main tube 63 in the internal space 76, there is mounted an L-shaped terminal 83 in the vicinity of the left end and it is constituted such that also the terminal 83 will slide along with the slide of the main tube 63. To the terminal 83, there is connected a sandwiching steering-member 7b and the sandwiching steering-member 7b is passed-through therein by passing a side portion of the main tube 63.

Consequently, the slide portion 100 is a portion for advancingly and retractingly steering the main tube 63 and concurrently, is a sort of sandwiching steering-lever which steers the sandwiching member 1 through the sandwiching steering-member 7b.

The terminal 83 moves through the main tube 63 along with the slide of the slide portion 100 which is this sandwiching steering-lever and the terminal 81 mentioned above moves through the steering-member 7c along with the slide of the sticking steering-lever 78, and at the respective movement terminal end positions of these terminal 81 and terminal 83, there are provided contact members 84, 85.

The contact members 84, 85 are connected with the connector portion 86 of the energy supply means 20 through the conductive wires d1, d2 and they are provided at mutually separated positions in the steering unit at hand 70, as shown in FIGS. 3 and 4. The reason why the contact members 84, 85 are installed separately in this manner is because a predetermined length X1 (see FIG. 4) is necessary as the working distance thereof caused by a fact that the sticking member 2 and the sandwiching member 1 carry out the sticking and the sandwiching operations by the movement in the axis direction of the steering-members 7c, 7b.

In particular, the contact members 84, 85 of this exemplified embodiment are constituted so as to contact with the terminals 81, 83 respectively before reaching the moving completed positions of the terminals 81, 83 which move along with the movement of the sticking steering-member 7c and the sandwiching steering-member 7b. Also, a switch SW (see FIG. 1) for on-off controlling electric current from the energy supply means 20 is provided at either one of the conductive wires d1 and d2. It should be noted that it is preferable for the switch SW to be provided at the steering unit at hand 70 itself or in the vicinity thereof so as to make it possible to be steered at hand easily, but it is also allowed for the switch to be a foot switch installed at feet.

The contact members 84, 85 will be explained in more detail. As shown in FIG. 4, the contact members 84, 85 include attachment portions S1 which contact with the terminals 81, 83, leg portions S2 protruded from the attachment portions S1, tubular collars S3 in which protrusion ends of the leg portions S2 are housed in the inside thereof and springs S4 for spring-biasing the leg portions S2 toward the outsides. Consequently, the respective attachment portions S1 are always protruded by the springs S4 and it happens that they are retreated when being pushed by the terminals 81, 83, so that they are constituted so as to possess conductable ranges X2 of predetermined lengths as shown in the drawing.

By doing like this, even if a sticking state of the sticking member 2 or a sandwiching state of the sandwiching member 1 is different depending on a fact that the thickness or the shape of the foramen ovale valve M2 or the like is different depending on persons and the moving completed positions of the terminals 81, 83 will become all different, the contact members 84, 85 contact with the terminals 81, 83 reliably, the electrical conduction becomes possible and reliability of the surgical procedure is secured. Also, there are also members having a constitution in which an electrical contact state is formed slidingly, but when comparing with such members, the contacts between the contact members 84, 85 and the terminals 81, 83 become reliable and furthermore, also troubles become fewer and also with respect to the slide operation of the terminals 81, 83, the frictional resistance force becomes less and lighter.

However, it is not necessary for all the groups of the contact members 84, 85 and the terminals 81, 83 to be in elastic contact states and it is allowed to employ the state at least for only one group thereof, and it is also allowed to employ a constitution in which the other one thereof is always in a contact state.

At a left end portion of the main body portion 75, as shown in FIG. 3, there is provided a coupling mechanism 90. The coupling mechanism 90 is a mechanism for making attachment and detachment of a Y-connector 72 easy with respect to the main body portion 75 and when engaging a flange portion f provided at a proximal portion of the Y-connector 72 with a ring shaped groove m formed on the main body portion 75, a pair of slide members 91 which are arranged to face each other (see FIG. 6) exert a pull-off stop function of the flange portion f and the Y-connector 72 becomes attachable and detachable.

It should be noted that it is preferable for the Y-connector 72 which can be injected with a contrast media or the like to be interlocked to the distal end of the steering unit at hand 70 by an interlock member 71, but in case of not using the Y-connector 72, it happens that the guiding catheter 31 having the flange portion f is to be directly interlocked to the main body portion 75. It is also allowed for the Y-connector 72 to be provided at an arbitrary position of the guiding catheter 31.

FIG. 6 is a schematic view of the coupling mechanism and here, in order to make the understanding thereof easier, one of the slide members 91 is shown by a solid line and the other is shown by a broken line.

The coupling mechanism 90 is a mechanism in which the slide members 91 which are a pair of flat plates with similar shapes are adjacently arranged mutually and both the slide members 91 are housed so as to be slidable in the direction perpendicular to the axis at the left end portion of the main body portion 75 such that they approach to each other by being spring-biased by means of spring members 92 at the distal ends. Consequently, when pushing pushes 93 of the end portions of both the slide members 91 toward the inner side against the spring members 92, a through-hole 94 with a size by which the flange portion f can pass therethrough is formed in the center and it is possible to pass-through the flange portion f, and when releasing the pushes, the through-hole 94 is narrowed by the spring members 92 and it is possible to hold the Y-connector 72, the guiding catheter 31 and the like so as not to be disengaged from the main body portion 75.

On the both sides of the side end regions of the main body portion 75, as shown in FIG. 3, there are provided in grooves 96 with a pair of guide bars 95 for advancing and retracting the slide portion 100 with respect to the main body portion 75. At one terminals of the guide bars 95, there are provided large diameter portions 97 for pull-off stop and they are constituted so as to stop by being attached to stopper members 98 provided at the outer end portions of the grooves 96.

The guide bars 95 are extended from the main body portion 75 toward the slide portion 100 and the end portions thereof are interlocked with the slide portion 100 by concave-convex engagement.

The main operation rod 7a passing-through the main tube 63 is extended until the outside thereof by passing-through a through-hole 101 formed in the center of the slide portion 100 and there is provided at an exit portion with a holding-portion locking mechanism 102.

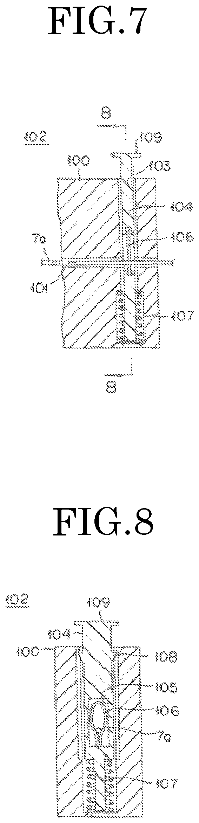

FIG. 7 is a cross-section view showing a holding-portion locking mechanism and FIG. 8 is a cross-section view along an 8-8 line in FIG. 7. The holding-portion locking mechanism 102 is provided at the slide portion 100 of the steering unit at hand 70, includes a rod grasping spring 106 for grasping the main operation rod 7a and a slide stick 104 for releasing the grasp by the rod grasping spring 106, and restricts the axis direction movement of the main operation rod 7a depending on elasticity by a holding portion 60 mentioned later by means of the slide stick 104 and the rod grasping spring 106; and it is constituted such that a lock state of continuing the holding of the biological tissue M by the holding portion 60 and an unlock state of releasing this lock state can be selected.

Specifically, there are included the slide stick 104 provided in a slide hole 103 perpendicular with respect to the axis line of the through-hole 101 through which the main operation rod 7a established at the slide portion 100 is passed, the rod grasping spring 106 which is fixedly installed in a spring holding hole 105 established approximately in the center of the slide stick 104 and which grasps the main operation rod 7a elastically, an auxiliary spring 107 for applying a protrusion behavior from the slide hole 103 to the slide stick 104, and a stopper member 108 for preventing this protrusion.

In this exemplified embodiment, the slide stick 104 is a plate shaped stick which moves upward and downward, and the rod grasping spring 106 is a spring having a shape in which the lower end in the drawing is narrow and the upper portion thereof is widened comparatively largely, but it is also allowed for such a slide stick 104 and a rod grasping spring 106 to be any kinds of members if they are a working member and a grasp member for performing the grasping and the releasing of the main operation rod 7a.

Consequently, the slide stick 104 is protruded outward by the auxiliary spring 107 on a steady basis and it becomes in a lock state in which the rod grasping spring 106 grasps the main operation rod 7a elastically, and when a push piece 109 provided at the top end of the slide stick 104 is depressed to the inner side against the auxiliary spring 107, this lock is released and it becomes in an unlock state in which the main operation rod 7a is in a free state. As a result thereof, if the main operation rod 7a is lock-unlock operated by the holding-portion locking mechanism 102 and, for example, if the holding portion 60 mentioned later is locked in a state of holding the foramen ovale valve M2, it is possible to maintain this holding state and if the lock is released, it becomes in a straight line shape automatically depending on the elasticity possessed by the holding portion 60 and the holding state of the foramen ovale valve M2 can be released.

The energy supply means 20 is means for supplying electric energy to the clamping means K and although the detailed explanation is omitted because of the well known system constitution thereof, it is preferable, regardless of considering a direct current power source or an alternate current power source, to employ electrical means. However, not only this but also any kind of means can be employed if the means is one which can supply energy which is capable of fusing the foramen ovale valve M2 and the atrial septum secundum M1 which are sandwiched by the clamping means K depending on the heat and capable of pressing and attaching them depending on an adhesive factor of collagen, elastin or the like. For example, it is also possible to use supersonic waves, lasers, microwaves, high frequencies or the like.

Also, as the electric energy supply system, it is possible to use such as a monopolar system in which electric conduction is executed between the sticking member 2 or the sandwiching member 1 on the right atrium R side and a counter-electrode plate provided on the surface of the body, a bipolar system in which electric conduction is executed between the sandwiching member 1 on the right atrium R side and the sticking member 2 on the left atrium L side and the like. In particular, if there is used a bipolar system in which the electric current is controlled by impedance of the biological tissue between the sticking member 2 and the sandwiching member 1, it is possible to have correspondence easily in response to a state of the tissues of the foramen ovale valve M2 and the atrial septum secundum M1 which are different depending on persons, and there can be obtained advantages of safety and usability of the surgical procedure.

FIG. 9 is a cross-section view along a 9-9 line in FIG. 2, in which the catheter 30 and the like are omitted. The holder 50 is, as shown in FIG. 9, established with five lumens L1 to L5, and in the first and second lumens L1, L2 and in the third and fourth lumens L3, L4, there are passed-through the sticking member 2 and the sandwiching member 1 as mentioned before. Also, in the fifth lumen L5 which is in the center and whose aperture diameter is maximum, there is passed-through the main tube 63 and here, there is also passed-through or housed the positioning hold means 60 mentioned later.

The positioning hold means 60, as shown in FIG. 2, generally includes a positioning portion 61 for positioning the sticking member 2 with respect to the foramen ovale O and a holding portion 62 for holding the foramen ovale valve M2 non-retractably with respect to the sticking direction of the sticking member 2, and it is housed in the guiding catheter 31 on a steady basis, but when used, as shown in the drawing, it is pushed out from the guiding catheter 31 by steering the main operation rod 7a and the main tube 63.

If explaining it in more detail, there are provided in the center lumen L5 with the main tube 63 and the main operation rod 7a which is provided freely advancingly and retractingly in the axis direction in the main tube 63. The main tube 63 is a tube for pulling-in and withdrawing the positioning hold means 60 into the catheter 30 other than is a tube having a function as the center axis mentioned above and also, it is a tube for reinforcing the catheter 30.

There is provided at the distal portion of the main tube 63 with the positioning hold means 60, and the positioning hold means 60 is constituted by the positioning portion 61 and the holding portion 62.

The positioning portion 61 is operated expandably and contractibly by the steering of the main operation rod 7a and is constituted by a pair of first elastic wires 66 for interlocking the main tube 63 and a middle sleeve body 64, and it is a portion for positioning the sticking member 2 with respect to the foramen ovale O.

The holding portion 62 includes a bump member 68 provided at a distal portion of the main operation rod 7a, a distal sleeve body 65 and a pair of second elastic wires 67 for interlocking the middle sleeve body 64 and the distal sleeve body 65; and it is a portion for holding the foramen ovale valve M2 by the bump member 68 and the distal sleeve body 65.

The positioning portion 61 protrudes the main operation rod 7a from the distal end of the main tube 63, displaces the first elastic wires 66 outward depending on the operation for advancing and retracting the main operation rod 7a in the axis direction, depresses the inner edge of the foramen ovale O with approximately equal elastic force by the respective first elastic wires 66, and center-aligns the sticking member 2 with respect to the foramen ovale O. In other words, there is exerted a function in which the sticking member 2 positioned between both the first elastic wires 66 is to be positioned at a central portion of the foramen ovale O.

The holding portion 62 includes a curving mechanism W for curving the distal portion of the main operation rod 7a by advancingly and retractingly steering the main operation rod 7a in the axis direction. The curving mechanism W curves the holding portion 62 such that the sticking member 2 makes the foramen ovale valve M2 face to the sticking direction and there is exerted a function for holding the foramen ovale valve M2. Here, the curving mechanism W is constituted by the middle sleeve body 64, the distal sleeve body 65, the second elastic wires 67 for interlocking both the sleeve bodies 64, 65 and the bump member 68.

The proximal ends of the first elastic wires 66 are welded to the distal end of the main tube 63 and the distal sides thereof are welded to the middle sleeve body 64. On the other hand, the proximal ends of the second elastic wires 67 are welded to the distal end of the middle sleeve body 64 and the distal sides thereof are welded to the distal sleeve body 65.

As an embodiment of the first and second elastic wires 66, 67, it is preferable to use a metal wire having an outer diameter of around 0.1mm to 0.5mm and made of such as stainless steel, nickel-titanium, superelastic alloy (for example, Ni-Ti alloy) and the like. In addition, it is also allowed to prevent the damage of the tissue by coating a resin (flexible) tube on the metal wire.

The holding portion 62 is constituted such that the first elastic wires 66 on the proximal side are curved prior to the second elastic wires 67 on the distal side, the positioning of the sticking member 2 is carried out, subsequently, the main operation rod 7a itself is deformed together with the bump member 68 and the distal sleeve body 65, the positioning portion 61 positions the sticking member 2 and thereafter, the foramen ovale valve M2 is to be held.

For this constitution, for example, it is possible to use also a method in which there is used a wire having higher stiffness materially on the second elastic wire 67 side compared with on the first elastic wire 66 side, a method in which there is formed an easily deformable portion by bend-deforming a portion of the first elastic wire 66 beforehand or the like and in which caused by the deformation of the easily deformable portion when a traction force is acted thereto, the first elastic wire 66 is curved prior to the second elastic wire 67 and a similar method like that.

When doing like this, only by exerting traction on the main operation rod 7a backward, the first elastic wire 66 on the proximal side is attached to the inner edge of the foramen ovale O and positioning of the sticking member 2 is carried out, and when exerting the traction further, the second elastic wire 67 on the distal side is protrusion-deformed in a circular arc shape toward the outside direction in the radial direction and it is possible to hold the foramen ovale valve M2 non-retractably such that it becomes easy for the sticking member 2 to stick.

Also, the main operation rod 7a is constituted to be rotatable by 360 degrees in the main tube 63 centering around the axis line. If the main operation rod 7a is rotatable by 360 degrees, it is possible to make the main operation rod 7a position-displaced rotatingly when the distal end of the main operation rod 7a is inserted until the vicinity of the foramen ovale O, and even if the state of the foramen ovale O is deformed variously, it is possible, regardless of the state of the shape thereof, to pass-through the distal end of the device into the foramen ovale O and it is possible to make the surgical procedure not only to be easy but also to be carried out speedily.

When advancing and retracting the slide portion 100 with respect to the main body portion 75, it is possible to pull-in the main tube 63 which is fixedly attached to the slide portion 100 into the center lumen L5 of the catheter 30 and along with this operation, it is possible to withdraw the whole positioning hold means 60 into the catheter 30.

For a material constituting the main tube 63, it is possible to use a deformable elastic material such as, for example, polyimide resin, polyurethane, PET, nylon, fluoride resin, polypropylene and the like.

Also, for the main operation rod 7a, if it is a rod of a fine hollow wire material and a rod having comparative stiffness, any kind of rod may be employed, but it is preferable to use a fine tube of such as, for example, stainless, Ni-Ti, titanium and the like.

With respect to the guiding catheter 31 of this exemplified embodiment, it is allowed for the distal end thereof to be curved gently in a circular arc shape such that it can be easily directed toward the foramen ovale O between the foramen ovale valve M2 and the atrial septum secundum M1. The foramen ovale valve M2 and the atrial septum secundum M1 are different depending on persons, so that when the distal end of the guiding catheter 31 is curved, it can be directed toward the foramen ovale O only by rotating the guiding catheter 31 itself and safety and usability of the surgical procedure are improved compared with a case of a straight line shape.

Next, the operation of this exemplified embodiment will be explained.

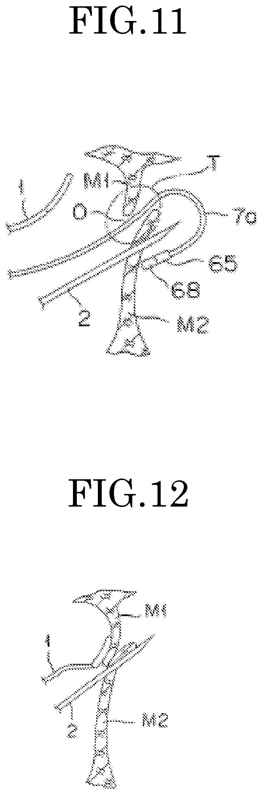

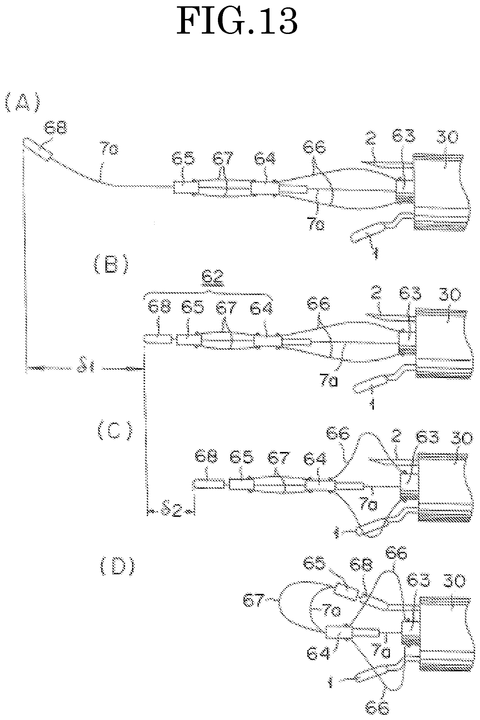

FIG. 10 is a cross-section schematic view in which a main operation rod is inserted into the foramen ovale, FIG. 11 is a cross-section schematic view of a state in which the foramen ovale valve is held and a sticking member is stuck, FIG. 12 is a cross-section schematic view in which the foramen ovale valve and the atrial septum secundum are sandwiched by a sticking member and a sandwiching member, and FIGS. 13A to 13D are schematic views showing operation states of a distal portion of the PFO closing device. It should be noted in FIGS. 13A to 13D that the shapes and the positions of the second elastic wires 66 are in approximately the same-plane states with respect to the sandwiching member 1 and the sticking member 2, but the illustrated positions are shown, in order to make it easy to understand, by the states displaced by 90 degrees and they are different from the actual deformation states.

First, a surgery operator retreats the slide portion 100 of the steering unit at hand 70 with respect to the main body portion 75 and a state is made in which the sandwiching member 1, the sticking member 2 and the like are housed in the guiding catheter 31. In this state, by using a guide wire as a guide, the distal end of the guiding catheter 31 is inserted from a predetermined position of a living body and is made to reach until the right atrium R through the femoral vein J*.* It should be noted that it is also allowed to insert only the guiding catheter 31 into a living body and thereafter, to insert the catheter 30 by using that as a guide.

When the distal end of the guiding catheter 31 reaches the right atrium R, the slide portion 100 is moved forward a little bit, and the main tube 63 is moved, the catheter 30 is pushed out from the guiding catheter 31 and is directed toward the foramen ovale O between the foramen ovale valve M2 and the atrial septum secundum M1. The distal end of the guiding catheter 31 is curved, so that the catheter 30 is guided by the guiding catheter 31 and is directed toward the foramen ovale O comparatively easily.

Next, the push piece 109 of the holding-portion locking mechanism 102 is pushed and the main operation rod 7a is made to be in an unlock state, and the main operation rod 7a is moved forward and, as shown in FIG. 13A, the distal end of the main operation rod 7a is protruded from the distal sleeve body 65 and inserted into the left atrium L. It is possible to observe this protruding state according to an X-ray image from the outside by eyes if a radiopaque marker is provided on the bump member 68 or the like, and it is possible to sensuously identify the position of the distal end of the main operation rod 7a when the distal end of the main operation rod 7a is bumped with an inner wall of the left atrium L or the like according to this protrusion even in a case in which it is difficult to observe by eyes. In the present exemplified embodiment, the main operation rod 7a is made to be rotatable by 360 degrees, so that, as shown in FIG. 10, the main operation rod 7a can be moved forward while rotating and it is possible to insert it into the foramen ovale O easily.

After the identification of the distal position of the main operation rod 7a, as shown in FIG. 13B, the main operation rod 7a is moved backward until the bump member 68 of the main operation rod 7a attaches to the distal sleeve body 65 (amount of backward movement is "δ1" in FIG. 13B). Then, the main body portion 75 is steered, and the second elastic wire 67, the sandwich member 1 and the sticking member 2 are positioned in the vicinity of the foramen ovale valve M2 and the whole holding portion 62 is inserted into the left atrium L side.

When the main operation rod 7a is further moved backward (amount of backward movement is "δ2" in FIG. 13C), the operation force for the backward movement is transmitted by the main operation rod 7a to the first elastic wire 66 firmly fixed on the distal end of the main tube 63 through the bump member 68, the distal sleeve body 65, the second elastic wire 67 and the middle sleeve body 64, and the first elastic wire 66 is, as shown in FIG. 13C, protruded and deformed in an arc shape toward the outside direction in the radial direction. However, at this time point, the second elastic wire 67 is not deformed.

Consequently, it happens that the first elastic wire 66 is deformed while pushing and widening an opening edge portion of the foramen ovale O, so that the sticking member 2 which is provided in close vicinity of the first elastic wire 66 is center-aligned with respect to the foramen ovale O and the sticking member 2 is positioned at the center of the foramen ovale O.

Further, when the main operation rod 7a is retreat-steered and, as shown in FIG. 13D, when a rear end of the middle sleeve body 64 is attached to the distal end of the main tube 63, the first elastic wire 66 is not deformed so much and the second elastic wire 67 of the distal side is protruded and deformed in an arc shape toward the outside direction in the radial direction by the operation force. Consequently, as shown in FIG. 11, in the left atrium L, the bump member 68 and the distal sleeve body 65 are curved so as to approach to the sticking member 2, so that it happens that the bump member 68 and the distal sleeve body 65 are attached to the surface of the left atrium side of the foramen ovale valve M2 and hold this.

In this valve holding state, if the main operation rod 7a is locked by the rod grasping spring 106 of the holding-portion locking mechanism 102, the position of the main operation rod 7a is locked. Therefore, even if the surgery operator releases his hand from the main operation rod 7a, the holding state of the foramen ovale valve M2 is maintained and it never happens that the holding is loosened. As a result thereof, it is not necessary for the surgery operator, for example, to hold down the steering unit at hand 70 by his left hand, it becomes possible for him to move the sticking steering-lever 78 forward in the concave groove 77 only by his right hand, to protrude the sticking member 2 from the distal end of the catheter 30 through the terminal 81 and the steering-member 7c and to stick the sticking member 2 into a predetermined position of the foramen ovale valve M2. There never happens such a situation in which the sticking is impossible caused by the looseness of the holding of the foramen ovale valve M2.

The position of the sticking member 2 is determined by the positioning hold portion 60, so that there is no fear of deviation and also, when the sticking member 2 is stuck once, the position of the sticking member 2 becomes a position fixed in relation to the foramen ovale valve M2. Therefore, it becomes easy for the surgery operator to perform the sticking operation.

In the steering unit at hand 70, it becomes, by this sticking, in a state in which the terminal 81 contacts with the contact member 84 elastically.

When the sticking is completed, the slide portion 100 is moved forward further with respect to the main body portion 75 and when the main tube 63 is moved forward, the flat board portion 1a of the sandwiching member 1 protrudes from the distal end of the catheter 30 through the steering-member 7b. Then, when the flat board portion 1a becomes in a position facing the atrial septum secundum M1, the slide portion 100 is retreated a little bit from the main body portion 75. Thus, the flat board portion 1a is displaced so as to approach to the sticking member 2 and depresses the atrial septum secundum M1 toward the foramen ovale valve M2, and the atrial septum secundum M1 and the foramen ovale valve M2 are fixed in the thickness direction, that is, the positions thereof in the forward-backward direction are fixed in the operation state and, as shown in FIG. 12, it becomes in a state in which the atrial septum secundum M1 and the foramen ovale valve M2 exist between the sandwich member 1 and the sticking member 2.

In this stage, if the push piece 109 of the holding-portion locking mechanism 102 is pushed and the lock of the main operation rod 7a by the rod grasping spring 106 is released, pressurization of the first elastic wire 66 and the second elastic wire 67 by the main operation rod 7a and the bump member 68 disappears, the first elastic wire 66 and the second elastic wire 67 are lengthened in straight line shapes by the elastic forces themselves, and they become in a state shown in FIG. 13B. Consequently, when steering the slide portion 100 retreatingly, the whole positioning hold means 60 is retreated through the main tube 63 and withdrawn in the lumen L5 of the catheter 30.

Also, it happens that the retreat steering of this slide portion 100 makes the bend portion 1c of the sandwiching member 1 to be deformed so as to approach to the sticking member 2 side from the end portion of the holder 50 through the steering-member 7b, so that as shown in FIG. 12, the atrial septum secundum M1 and the foramen ovale valve M2 are sandwiched strongly between the sandwiching member 1 and the sticking member 2. On the other hand, in the steering unit at hand 70, also the terminal 83 mounted on the main tube 63 is retreated and contacts with the contact member 85.

In other words, it happens that the retreat of the slide portion 100 in this stage will handle the sandwiching of the biological tissues M and the contact state of the terminal 83 and the contact member 85 at one blow. Furthermore, the terminal 81 and the contact member 84 became in a contact state previously, so that it becomes in a state in which it is possible to supply electric energy to the sandwiching member 1 and the sticking member 2 depending on the contact of the terminal 83 and the contact member 85.

Consequently, when the surgery operator turns on the switch SW, predetermined electric energy which is controlled by the control unit 22 is supplied to the sandwiching member 1 and the sticking member 2 through the steering-members 7b, 7c, and the atrial septum secundum M1 and the foramen ovale valve M2 are heated.

The control unit 22 controls the output power to be low and makes it difficult for the thrombus to be attached, so that even if a portion of the sandwiching member 1 and the sticking member 2 is exposed in the blood, it is possible to prevent the thrombus from being attached to the sandwiching member 1 and the sticking member 2. However, if coating for suppressing thermal conduction is applied to the surfaces, which are exposed to the blood, of the sandwiching member 1 and the sticking member 2, it happens that the attachment of the thrombus is prevented reliably furthermore and it is preferable.

When continuing the heating while maintaining the fusing temperature, the tissues of the atrial septum secundum M1 and the foramen ovale valve M2 melt and are fused mutually by a bonding factor of collagen, erastin or the like.

When the fusion is completed, the switch SW is turned off, the supply of the electric energy is stopped, the slider portion 100 is steered advancingly and retractingly, and the sandwiching member 1 and the sticking member 2 are housed in the guiding catheter 31 through the main tube 63. Then, when the main body portion 75 is retreated so as to be away from the living body, the guiding catheter 31 is pulled out from the living body and the surgical procedure is completed. It should be noted that after the completion of the surgical procedure, a very small hole remains in the foramen ovale valve M2 caused by the pulling-out of the sticking member 2, but it will be healed afterward and it never happens that a bad influence such as occurrence of the thrombus or the like occurs.

### <Second Exemplified Embodiment>

With respect to this exemplified embodiment, in the steering unit at hand 70, a portion for controlling the supply of the electric energy and a portion of the steering-lever for steering the sandwiching member 1 and the sticking member 2 are different from those of the first exemplified embodiment.

In the first exemplified embodiment, the on-off control of the electric energy supply is carried out by a switch SW, but it is constituted such that this exemplified embodiment does not use a switch SW and the control is carried out by attachment-detachment operation of the sticking steering-lever 78 and a sandwiching steering-lever 100A which are provided at the steering unit at hand 70 with respect to connection members 111 of the electric energy supply means 20 side.

Also, it is constituted in the first exemplified embodiment such that there is provided at the main body portion 75 of the steering unit at hand 70 with the sticking steering-lever 78 which steers the sticking member 2 on the upper surface thereof and the sandwiching member 1 is steered by the slide portion 100, but there are provided, in this exemplified embodiment, with the sticking steering-lever 78 and the sandwiching steering-lever 100A so as to protrude from the upper and lower surfaces of the main body portion 75. It should be noted that the same reference numerals are applied for the members which are in common with those of the first exemplified embodiment mentioned above and the explanation thereof will be omitted.

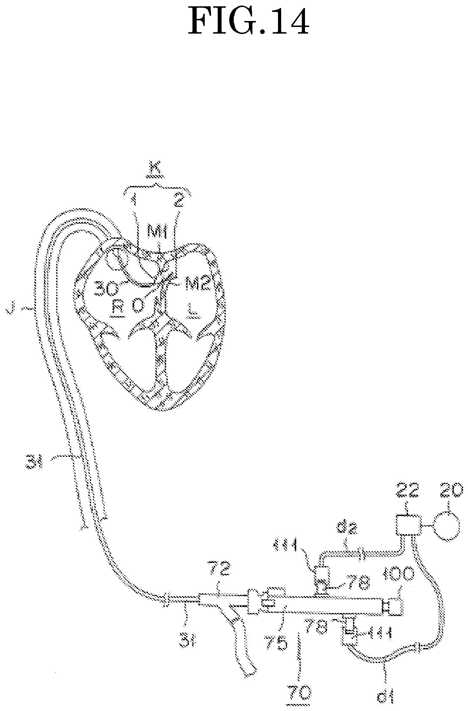

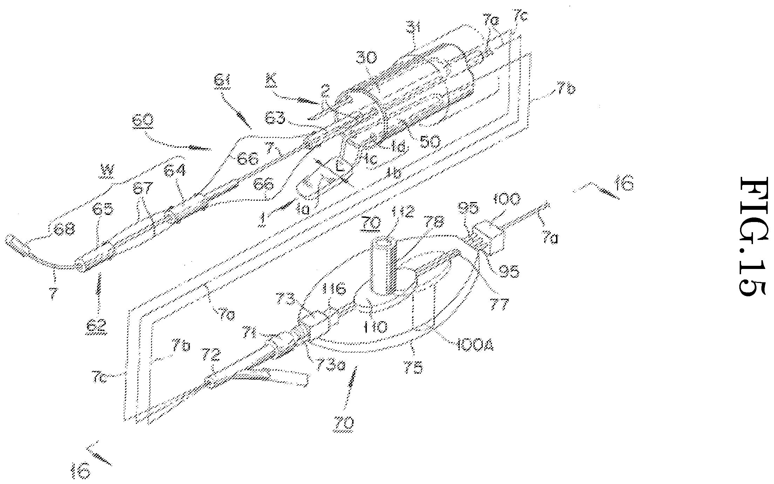

With respect to the steering unit at hand 70 of this exemplified embodiment, as shown in FIG. 14 and FIG. 15, in order to make it possible for the surgery operator to execute the gripping by one hand, there is protruded, on the front surface side (upper surface side) of the main body portion 75 which was formed flatly, the sticking steering-lever 78 with which the proximal side of the sticking steering-member 7c is interlocked, and there is protruded, on the rear surface side (lower surface side) thereof, the sandwiching steering-lever 100A with which the proximal side of the steering-member 7b of the sandwiching member 1 is interlocked.

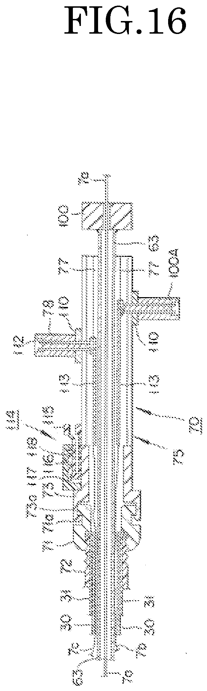

The respective steering-levers 78, 100A are, as shown in FIG. 14 and FIG. 16, provided slidably in the concave groove 77 which is formed in the main body portion 75, and it is constituted such that they can slidingly move smoothly without toppling depending on flange portions 110 provided at lower end portions of the respective steering-levers 78, 100A. Also, at the end portions of the sticking steering-lever 78 and the sandwiching steering-lever 100A, there are formed concave portions 112 and it is constituted, by the attachment and detachment of the concave portions 112 and the connection members 111 of the electric energy supply means 20, such that the on-off control of the electric energy supply is carried out.

Consequently, if the surgical procedure by both the steering-levers 78, 100A is carried out and the concave portions 112 of both the steering-levers 78, 100A are connected to the connection members 111 while maintaining the state of sandwiching the biological tissue M, it is possible to supply the electric energy, so that it is possible to realize an electric energy supply at any desired time point and also, it never happens that the conductive wires d1, d2 become obstacle during the surgical procedure, and usability and reliability of the steering are improved drastically. Furthermore, it never happens that the electric energy is supplied to the biological tissue M unnecessarily, so that it is possible to prevent also the attachment of the thrombus.

It is also allowed for the respective steering-members 7b, 7c and the respective steering-levers 78, 100A to be connected directly by soldering or the like, but it is constituted in this exemplified embodiment such that the proximal sides of the respective steering-members 7b, 7c are soldered on the distal sides of slide pieces 113 which slide in the concave groove 77 formed in the main body portion 75, electrical conductors provided on the respective steering-levers 78, 100A are soldered on the proximal sides of the slide pieces 113, and these electrical conductors are introduced until the concave portions 112 and become in an conduction state electrically with respect to the connection member 111. If connection is realized electrically through the slide piece 113 in this manner, it happens that not only steerability of the device is improved but also strength or durability thereof is improved and it is preferable.

Also, the steering unit at hand 70 of this exemplified embodiment includes, at the distal portion of the main body portion 75, a sticking-member locking mechanism 114 which locks a state in which the sticking member 2 is protruded from the distal portion of the catheter 30.

The sticking-member locking mechanism 114 is a mechanism in which there is provided, on the distal side of the concave groove 77 formed in the main body portion 75, with a bump member 115 for restricting the slide movement of the sticking steering-lever 78 and which locks the slide movement by pressing the sticking steering-lever 78 onto the bump member 115. When the sticking steering-lever 78 is pressed onto the bump member 115 in a state in which the sticking member 2 is protruded from the distal portion of the catheter 30, the position of the sticking steering-lever 78 can be locked and a protrusion state of the sticking member 2 can be maintained. Consequently, it is possible to supply the electric energy while pressing the sticking steering-lever 78 onto the bump member 115, it never happens that the sticking member 2 is deviated when supplying the electric energy, and it becomes possible to carry out the surgical procedure more stably and also more accurately.

As an illustrative embodiment of the sticking-member locking mechanism 114, as shown in FIG. 16, it is a mechanism in which a tubular block 116 is provided fixedly at the main body portion 75 and the bump member 115 is provided position-adjustably in the tubular block 116. As position adjustment means of the bump member 115, a constitution is employed in which a plurality of protrusions 117 having non-retractable shapes are formed in the inner surface of the tubular block 116 and claw portions 118 on the side of the bump member 115 of the structure having elasticity in the radial direction is latched to the protrusion 117.

At the rear end portion of the main body portion 75, the slide portion 100 is provided through two pieces of the guide bars 95 similarly as the first exemplified embodiment and it is constituted such that the main tube 63 will be advanced and retracted by the slide portion 100, but in this exemplified embodiment, the holding-portion locking mechanism 102 is not provided. It is needless to say that it is also allowed to provide the holding-portion locking mechanism 102 here.

At the distal end of the main body portion 75, the Y-connector 72 into which a contrast media or the like can inject is interlocked through the interlock members 71, 73. The proximal portion of the interlock member 71 is formed with a groove portion, and this groove portion 71a and a claw portion 73a of the interlock member 73 are engaged and interlocked. The distal portion of the interlock member 71 is screwed by the proximal portion of the Y-connector 72 and the proximal end of the catheter 30 and the end portion of the guiding catheter 31 are sandwiched between the interlock member 71 and the Y-connector 72.

Next, the operation of this exemplified embodiment will be explained, in which the disclosure of the aspects similar to those of the first exemplified embodiment will be omitted and different portions there-between will be described.

After adjusting the position of the bump member 115 of the sticking-member locking mechanism 114, the sticking member 2 is stuck to the biological tissue M held by the positioning hold means 60. Depending on this sticking, the sticking steering-lever 78 is attached to the sticking-member locking mechanism 114, so that when holding this attachment state, the position of the sticking steering-lever 78 is locked and there is no fear that the position of the sticking member 2 is deviated. Consequently, while holding the sticking steering-lever 78, the surgery operator steers the sandwiching steering-lever 100A provided on the lower surface side of the steering unit at hand and executes the sandwiching of the biological tissue M.

With respect to the sandwiching of the biological tissue, first, the sandwiching steering-lever 100A is moved forward and the sandwiching member 1 is made to protrude from the distal end of the catheter 30 through the steering-member 7c. Thus, as shown in FIG. 11, it becomes in a state in which the atrial septum secundum M1 and the foramen ovale valve M2 exist between the sandwiching member 1 and the sticking member 2.

Then, when the sandwiching steering-lever 100A is made to retreat, the sandwiching member 1 retreats through the steering-member 7c, and the atrial septum secundum M1 and the foramen ovale valve M2 are sandwiched strongly between the sandwiching member 1 and the sticking member 2.

In a state in which the forward position of the sticking steering-lever 78 and the retreat position of the sandwiching steering-lever 100A are maintained, if the connection members 111 are fitted in a concave-convex form with the concave portions 112 of the respective steering-levers 78, 100A, a predetermined electric current controlled by the control unit 22 flows from the sandwiching steering-lever 100A and the sticking steering-lever 78 to the sandwiching member 1 and the sticking member 2 through the steering-members 7b, 7c, and the atrial septum secundum M1 and the foramen ovale valve M2 are heated and fused.

When the fusion is completed, the electrical conduction is stopped if disengaging the connection between the connection member 111 and either one of the steering-levers 78, 100A.

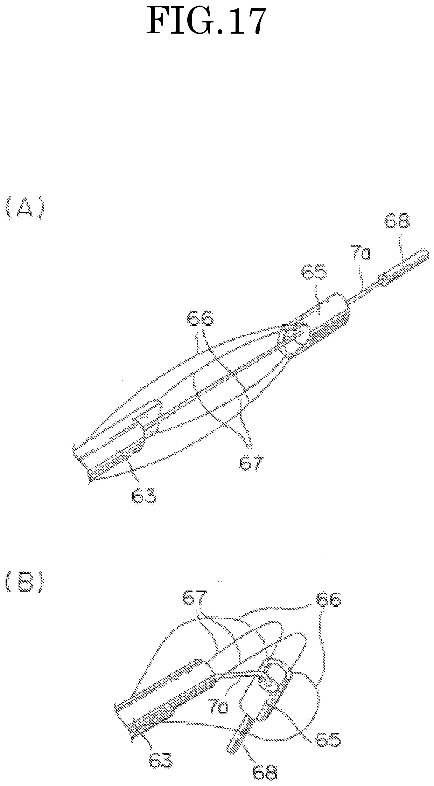

The present invention is not limited only by the exemplified embodiments mentioned above and it is possible for a person skilled in the art to employ various modifications within the technical concept of the present invention. For example, the positioning hold means 60 in the exemplified embodiment mentioned above includes the middle sleeve body 64, but the present invention is not limited only by this. For example, FIG. 17 is a schematic view showing another example of the positioning hold mechanism and, as shown in FIG. 17A, it is also allowed to provide the first elastic wire 66 and the second elastic wire 67 between the distal sleeve body 65 and the main tube 63 without providing the middle sleeve body 64 which is provided in the present exemplified embodiment. In this exemplified embodiment, when the main operation rod 7a is moved backward, as shown in FIG. 17B, the second elastic wire 67 is bent and deformed into an arc shape while the first elastic wire 66 is protruded and deformed in an arc shape in the radial direction toward the outside direction. More specifically, it happens that the positioning of the sticking member 2 to the center of the foramen ovale O caused by the first elastic wire 66 and the holding of the foramen ovale valve M2 caused by the bump member 68 and the distal sleeve body 65 which are bent by the second elastic wire 67 are to be performed simultaneously based on one action caused by the backward movement of the main operation rod 7a.

In the exemplified embodiment mentioned before, it was explained with respect to a device which is used for the medical treatment for closing the defect of the PFO, but the present invention is not limited only by this and it is also possible to use it in a case in which a pathway shaped defect referred to as a left auricle closing device (Left Atrial Appendage) is closed or in a case in which the biological tissue of a predetermined region is necrotized thermally.

With respect to the PFO closing device of aforesaid exemplified embodiment, it is merely housed in the catheter and steers the clamping means by the steering-member, but it is not limited only by this and, for example, it is also possible to carry it until a predetermined position by employing a combination with a so-called catheter having a balloon.

### INDUSTRIAL APPLICABILITY

The present invention can be utilized for a medical device which can close a defect of a biological tissue simply and also safely.

### BRIEF DESCRIPTION OF DRAWINGS

FIG. 1 is a schematic cross-section view showing a medical device relating to a first exemplified embodiment of the present invention;
FIG. 2 is a perspective view of a main portion showing one example of the same device;
FIG. 3 is a cross-section view along a 3-3 line in FIG. 2;
FIG. 4 is a schematic view showing contact members and terminals;
FIG. 5 is a vertical cross-section view showing a sticking steering-lever portion in FIG. 3;
FIG. 6 is a schematic view of a coupling mechanism;
FIG. 7 is a cross-section view showing a holding-portion locking mechanism;
FIG. 8 is a cross-section view along an 8-8 line in FIG. 7;
FIG. 9 is a cross-section view along a 9-9 line in FIG. 2;
FIG. 10 is a cross-section schematic view in which a main operation rod is inserted into a defect of a biological tissue;
FIG. 11 is a cross-section schematic view of a state in which a biological tissue is held and a sticking member is stuck thereto;
FIG. 12 is a cross-section schematic view in which biological tissues are sandwiched by a sticking member and a sandwich member;
FIG. 13A to FIG. 13D are schematic views showing operation states of the medical device;
FIGS. 14 are schematic perspective views showing another example of the positioning hold means, in which FIG. 14A shows a state at a normal time and FIG. 14B shows a state when holding the positioning;
FIG. 15 is a schematic cross-section view showing a medical device relating to another exemplified embodiment of the present invention;
FIG. 16 is a cross-section correspondence view along a 16-16 line in FIG. 15; and
FIGS. 17 are schematic views showing another example of a positioning hold mechanism, in which FIG. 17A shows a state at a normal time and FIG. 17B shows a state when holding the positioning.

## Claims

1. A medical device comprising:
clamping means (K) including a sticking member (2) for sticking a biological tissue which lies at a periphery of a defect existing in a biological tissue and a sandwiching member (1) for sandwiching said biological tissue so as to close said defect by cooperating with said sticking member (2);
a catheter (30) into which said sticking member (2) and said sandwiching member (1) are inserted;
electric energy supply means (20) for supplying electric energy to said sticking member (2) and said sandwiching member (1) of said clamping means; and
a steering unit at hand (70) mounted with a proximal portion of said catheter (30) for steering said clamping means, wherein said medical device is configured for closing said defect by supplying electric energy from said electric energy supply means and by joining said biological tissues mutually in a state of sandwiching said biological tissue by said sticking member (2) and
said sandwiching member (1), **characterised in that** said steering unit at hand (70) includes:
contact members (84, 85) which are connected to the other terminal portions of conductive wires (d1, d2) whose one terminals (81, 83) are connected to said electric energy supply means and which are provided at mutually separated positions,
terminals (81, 83) configured to be respectively positionable at respective steering-members which move said sticking member (2) and said sandwiching member (1), and
a control unit for on-off controlling the supply of said electric energy, wherein
said respective terminals (81, 83) are configured to contact with said contact members (84, 85) respectively along with the move of said respective steering-members and wherein said medical device is also constituted so as to supply said electric energy to said biological tissue between said sticking member (2) and sandwiching member (1) by turning on said control unit.

2. The medical device according to claim 1, **characterized in that** said control unit is constituted by a switch (SW) provided on said steering unit at hand (70) or in a vicinity thereof.

3. The medical device according to claim 1, **characterized in that** said control unit attaches and detaches at least one of steering-levers which steer said sticking member (2) and said sandwiching member (1) provided on said steering unit at hand (70) with respect to a connection member connected to said electric energy supply means electrically.

4. The medical device according to any one of claims 1 to 3, **characterized in that** either one group of said contact member and said terminal contacting mutually is made to be always in a contact state.

5. The medical device according to any one of claims 1 to 3, **characterized in that** at least one of said contact member and said terminal contacting mutually includes: an attachment portion for contacting with a contacting partner; a leg portion (S2) protruding from said attachment portion; and a tubular collar for protruding said leg portion housed in the inside toward the outside by a spring (7), wherein it is constituted such that the moving completed position of said terminal exists within the region in which said leg portion protrudes outward by the spring (7).

## Patentansprüche

1. Medizinische Vorrichtung, umfassend:
eine Klemmeinrichtung (K), die ein Stechelement (2) zum Einstechen in ein biologisches Gewebe, welches in einem Außenbereich eines in einem biologischen Gewebe vorhandenen Defekts liegt, und ein Zusammenpresselement (1) zum Zusammenpressen des biologischen Gewebes aufweist, um den Defekt durch Zusammenwirken mit dem Stechelement (2) zu verschließen;
einen Katheter (30), in den das Stechelement (2) und das Zusammenpresselement (1) eingesetzt sind;
eine elektrische Energieversorgungseinrichtung (20) zum Zuführen elektrischer Energie zu dem Stechelement (2) und dem Zusammenpresselement (1) der Klemmeinrichtung; und
eine im Griff angeordnete Lenkeinheit (70), die an einem proximalen Abschnitt des Katheters (30) zum Lenken der Klemmeinrichtung angebracht ist, wobei die medizinische Vorrichtung konfiguriert ist, um den Defekt durch Zuführen von elektrischer Energie aus der elektrischen Energieversorgungseinrichtung und durch Verbinden der biologischen Gewebe miteinander in einem Zustand des Zusammenpressens des biologischen Gewebes durch das Stechelement (2) und das Zusammenpresselement (1) zu verschließen, **dadurch gekennzeichnet, dass** die im Griff angeordnete Lenkeinheit (70) umfasst:
Kontaktelemente (84, 85), die mit den anderen Anschlussabschnitten von leitenden Drähten (d1, d2) verbunden sind, deren eine Anschlüsse (81, 83) mit der elektrischen Energieversorgungseinrichtung verbunden sind und die an voneinander getrennten Positionen vorgesehen sind,
Anschlüsse (81, 83), die konfiguriert sind, um jeweils an entsprechenden Lenkungselementen positionierbar zu sein, die das Stechelement (2) und das Zusammenpresselement (1) bewegen, und
eine Steuereinheit zur Ein-Aus-Steuerung der Zufuhr der elektrischen Energie, wobei die jeweiligen Anschlüsse (81, 83) konfiguriert sind, um mit den Kontaktelementen (84, 85) jeweils zusammen mit der Bewegung der jeweiligen Lenkungselemente in Kontakt zu treten, und wobei die medizinische Vorrichtung ebenfalls so ausgebildet ist, dass sie die elektrische Energie dem biologischen Gewebe zwischen dem Stechelement (2) und dem Zusammenpresselement (1) durch Einschalten der Steuereinheit zuführt.

2. Medizinische Vorrichtung nach Anspruch 1, **dadurch gekennzeichnet, dass** die Steuereinheit aus einem Schalter (SW) besteht, der an der im Griff angeordneten Lenkeinheit (70) oder in deren Nähe vorgesehen ist.

3. Medizinische Vorrichtung nach Anspruch 1, **dadurch gekennzeichnet, dass** die Steuereinheit sich mit mindestens einem der Lenkhebel verbindet und trennt, die das an der im Griff angeordneten Lenkeinheit (70) vorgesehene Stechelement (2) und das Zusammenpresselement (1) in Bezug auf ein mit der elektrischen Energieversorgungseinrichtung elektrisch verbundene Verbindungselement lenken.

4. Medizinische Vorrichtung nach einem der Ansprüche 1 bis 3, **dadurch gekennzeichnet, dass** eine von beiden der Gruppe von dem Kontaktelement und dem Anschluss zum gegenseitigen Kontaktieren so gestaltet ist, dass sie sich immer in einem Kontaktzustand befindet.

5. Medizinische Vorrichtung nach einem der Ansprüche 1 bis 3, **dadurch gekennzeichnet, dass** mindestens eines von dem Kontaktelement und dem Anschluss zum gegenseitigen Kontaktieren umfasst: einen Verbindungsabschnitt zum Kontaktieren mit einem Kontaktpartner; einen Schenkelabschnitt (S2), der aus dem Verbindungsabschnitt hervorsteht; und eine rohrförmige Manschette zum Hervorstehen des Schenkelabschnitts, der im Inneren untergebracht ist, in Richtung der Außenseite durch eine Feder (7), wobei sie derart ausgebildet ist, dass die Bewegungsabschlussposition des Anschlusses sich innerhalb des Bereichs befindet, mit dem der Schenkelabschnitt durch die Feder (7) nach außen hervorsteht.

## Revendications

1. Dispositif médical comprenant :
des moyens de clampage (K) comprenant un élément d'ancrage (2) pour se piquer dans un tissu biologique qui se trouve au niveau d'une périphérie d'une anomalie existant dans un tissu biologique et un élément de prise en sandwich (1) pour prendre en sandwich ledit tissu biologique de manière à fermer ladite anomalie en coopérant avec ledit élément d'ancrage (2) ;
un cathéter (30) dans lequel ledit élément d'ancrage (2) et ledit élément de prise en sandwich (1) sont insérés ;
des moyens d'alimentation en énergie électrique (20) pour alimenter en énergie électrique ledit élément d'ancrage (2) et ledit élément de prise en sandwich (1) desdits moyens de clampage ; et
une unité de direction à commande manuelle (70) montée avec une partie proximale dudit cathéter (30) pour diriger lesdits moyens de clampage, où ledit dispositif médical est conçu pour fermer ladite anomalie en appliquant de l'énergie électrique provenant desdits moyens d'alimentation en énergie électrique et en reliant lesdits tissus biologiques mutuellement dans un état où ledit tissu biologique est pris en sandwich par ledit élément d'ancrage (2) et par ledit élément de prise en sandwich (1),
**caractérisé en ce que** ladite unité de direction à commande manuelle (70) comprend :
des éléments de contact (84, 85) qui sont reliés aux autres parties de borne de fils conducteurs (d1, d2) dont les bornes (81, 83) sont connectées auxdits moyens d'alimentation en énergie électrique et qui sont placées en des positions mutuellement séparées, les bornes (81, 83) étant conçues pour pouvoir être positionnées respectivement au niveau d'éléments de direction respectifs qui déplacent ledit élément d'ancrage (2) et ledit élément de prise en sandwich (1), et
une unité de commande pour mettre en marche-couper ladite alimentation en énergie électrique, où lesdites bornes respectives (81, 83) sont conçues pour venir en contact avec lesdits éléments de contact (84, 85) respectivement en même temps que le déplacement desdits éléments de direction respectifs et où ledit dispositif médical est également conçu de manière à appliquer ladite énergie électrique audit tissu biologique entre ledit élément d'ancrage (2) et ledit élément de prise en sandwich (1) en mettant en marche ladite unité de commande.

2. Dispositif médical selon la revendication 1, **caractérisé en ce que** ladite unité de commande est constituée par un commutateur (SW) prévu sur ladite unité de direction à commande manuelle (70) ou à proximité de celle-ci.

3. Dispositif médical selon la revendication 1, **caractérisé en ce que** ladite unité de commande attache et détache au moins l'un des leviers de direction qui dirigent ledit élément d'ancrage (2) et ledit élément de prise en sandwich (1) prévus sur ladite unité de direction à commande manuelle (70) par rapport à un élément de connexion connecté électriquement auxdits moyens d'alimentation en énergie électrique.

4. Dispositif médical selon l'une quelconque des revendications 1 à 3, **caractérisé en ce que** l'un ou l'autre du groupe dudit élément de contact et de ladite borne en contact mutuel est fait pour être toujours dans un état de contact.

5. Dispositif médical selon l'une quelconque des revendications 1 à 3, **caractérisé en ce qu'**au moins l'un dudit élément de contact et de ladite borne en contact mutuel comprend : une partie de fixation pour venir en contact avec un élément de contact associé ; une partie branche (S2) faisant saillie depuis ladite partie de fixation ; et un collier tubulaire pour faire avancer vers l'extérieur ladite partie branche logée à l'intérieur au moyen d'un ressort (7), où il est conçu de telle sorte que la position de déplacement complet de ladite borne existe à l'intérieur de la région dans laquelle ladite partie branche fait saillie vers l'extérieur sous l'action du ressort (7).
